# EUROPÄISCHE PATENTSCHRIFT

(11) **EP 3 609 383 B1**
(45) Veröffentlichungstag und Bekanntmachung des Hinweises auf die Patenterteilung: **06.05.2026**
(21) Anmeldenummer: 18717590.6
(22) Anmeldetag: 11.04.2018
(51) Int. Cl.: A61B 1/00, A61B 1/005, A61B 1/015, A61B 1/018, A61B 1/06, A61B 1/07, A61B 1/12, A61B 1/273, A61B 1/31

(54) **ENDOSKOPKOPF MIT SCHWENKBARER KAMERA- UND ARBEITSKANAL-EINHEIT**
ENDOSCOPE HEAD HAVING A PIVOTABLE CAMERA AND WORKING CHANNEL UNIT
TÊTE D'ENDOSCOPE À UNITÉ DE CAMÉRA ET À UNITÉ DE CANAL DE TRAVAIL PIVOTANTES

(30) Priorität: 12.04.2017 DE 102017107978
(43) Veröffentlichungstag der Anmeldung: 19.02.2020
(73) Patentinhaber: Bob, Konstantin, 69469 Weinheim (DE)
(72) Erfinder: Bob, Konstantin, 69469 Weinheim (DE)
(74) Vertreter: Winter, Brandl - Partnerschaft mbB
(86) Internationale Anmeldenummer: PCT/EP2018/059265
(87) Internationale Veröffentlichungsnummer: WO 2018/189230

(56) Entgegenhaltungen:
- EP-A2- 1 132 041
- DE-A1- 102013 224 683
- US-A1- 2002 099 267
- US-A1- 2005 234 526
- US-A1- 2009 231 419
- US-A1- 2011 028 790
- US-A1- 2012 041 264
- US-A1- 2014 107 417

## Beschreibung

Die vorliegende Erfindung betrifft einen (Endoskop-integralen) Endoskopkopf oder einen (Endoskop-separaten) Endoskopkopf-Aufsatz / Endoskopkopf der Endoskop-adaptiven Bauart, der jeweils zumindest eine Optik zur Bildübertragung, ein Leuchtmittel, und einen Arbeitskanal zur Führung von (minimalinvasiven) Werkzeugen und/oder zur Durchströmung von Medien aufweist. Weiter betrifft die Erfindung ein Endoskop mit einem solchen Endoskopkopf oder Endoskopkopf-Aufsatz.

### Hintergrund der Erfindung

Endoskope sind medizinische Arbeitsgeräte zur visuellen Exploration von Hohlräumen in einem Patientenkörper. Sie weisen grundsätzlich optische Einrichtungen am distalen, d.h. körperzugewandten Endoskopende (auch Endoskopkopf genannt) sowie optional einen Arbeitskanal auf, der sich ausgehend von einem proximalen (körperabgewandten) Endoskopabschnitt oder extrakorporalen Endoskopgriff durch einen (daran sich anschließenden) flexiblen/biegesteifen oder starren Endoskopschaft hindurch bis zum Endoskopkopf ersteckt und das extrakorporale Einführen und Verwenden eines medizinischen Instruments wie z.B. einer Zange, Schere, Nadel, Schlinge, Messer und dergleichen ermöglicht.

Derartige Endoskope können wahlweise mit zusätzlichen Fähigkeiten versehen werden, etwa indem am distalen Endoskopende/Endoskopkopf eine Kappe oder Hülse auf den Endoskopkopf radial außenseitig aufgesetzt wird, die mit bestimmten Funktionen/Funktionselementen versehen oder ausgerüstet ist, wodurch das Endoskop nicht nur zur Exploration und/oder als Zugang für therapeutische Anwendungen sondern auch selbst als ein minimalinvasives Instrument zur Ausführung eines chirurgischen Vorgangs genutzt werden kann. Alternativ hierzu ist es aber auch vorgesehen, spezielle Endoskope für ganz bestimmte medizinische Anwendungen mit derartigen Fähigkeiten integral auszustatten, wobei derartige Spezialanfertigungen jedoch nur für diese besondere Anwendung geeignet sind.

Verschiedene diagnostische und/oder therapeutische Verfahren erfordern beispielsweise eine Bildgebung und/oder bei Bedarf therapeutische Techniken am Gallen- und/oder Bauchspeicheldrüsengang sowie den Lebergängen des Patienten. Da die vatersche Papille, welche den gemeinsamen Austritt von Gallen- und Bauchspeicheldrüsengang in das Duodenum bildet, seitlich in das Duodenum hineinragt, sind herkömmliche prograde (in Endoskoplängsrichtung blickende) Endoskope für derartige Eingriffe ungeeignet, da nicht genügend Schwenkraum im Duodenum vorhanden ist, um deren prograde Optik sowie den Arbeitskanal in eine seitwärts blickende Position auszurichten.

### Stand der Technik

Aus dem Stand der Technik (bspw. der US 2010/228086 A) sind zu diesem Zweck speziell angefertigte Duodenoskope bekannt, welche eine laterale (seitwärts blickende) oder retrograde (rückblickende) Optik (auch "Seitoptik" genannt) sowie einen seitwärts gerichteten Arbeitskanal aufweisen. Am Ausgang des Arbeitskanals solcher Duodenoskope ist zudem in der Regel ein sogenannter Albarranhebel vorgesehen, der durch Verschwenken ein gezieltes Führen/Umlenken eines im Arbeitskanal geführten Werkzeugs ermöglicht. Durch die seitwärts gerichtete Anordnung der Funktionseinheiten am Endoskopkopf wird eine Bildgebung und Behandlung im Bereich des Duodenums unter optimaler Nutzung des zur Verfügung stehenden Raums ermöglicht.

Derartige Endoskope mit Seitoptik sind jedoch sehr aufwendig und teuer in ihrer Fertigung und werden deshalb bislang als wiederverwendbare Geräte entwickelt und hergestellt. Der gekrümmte Arbeitskanal solcher Endoskope sowie die komplexe und hinterschnittreiche Konstruktion des Albarranhebels haben sich in der Praxis zudem als nicht sterilisierbar erwiesen bzw. der Sterilisationsprozess hat sich als zu materialermüdend für die filigrane Konstruktion des Albarranhebels herausgestellt, sodass nach einem Eingriff mit einem solchen Duodenoskop nur eine Desinfektion möglich ist. Dies hat zur Folge, dass nach einem Eingriff ein Bakterienrasen (Biofilm) im Arbeitskanal und/oder dem Hilfskanal des Endoskops bestehen bleibt. Löst sich dieser Biofilm dann bei einem darauf folgenden Eingriff, etwa weil ein Instrument durch den Arbeitskanal geschoben wird, so kann er bspw. in den Gallengang gelangen und dort schwerwiegende Entzündungen bis hin zu einer Sepsis beim Patienten verursachen.

Weiter haben solche Geräte den Nachteil, dass sie nur für wenige, sehr spezifische Eingriffe im Bereich des Duodenums eingesetzt werden können, da weder die Optik noch der Arbeitskanal in prograde Richtung gerichtet werden können. Zudem ist die Navigation im Körper mit seitwärts blickenden Endoskopen im Allgemeinen eher schwierig, da ein Vorausblicken immer ein Abkrümmen des dem Endoskopkopf unmittelbar vorgeordneten "Deflectings" (aktiv abkrümmbarer Endoskopschaft-Abschnitt) um ca. 90° erfordert, wodurch wiederum mehr Platz in Endoskopquerrichtung benötigt wird, der nur im Magen vorhanden ist. Die Druckschriften DE 20 2013 007 416 U1, JP H10 258022 A, DE 10209986 A1, US 5413107 A und DE 10 2013224683 A1 offenbaren Beispiele für prograde, flexible Endoskope mit einem solchen vorstehend beschriebenen Deflecting. Bei einigen der zitierten Druckschriften ist der Endoskopkopf austauschbar am distalen Deflectingende angebracht.

Aus dem Stand der Technik sind, wie bspw. in der DE 10 2013 222 279 A1 oder der DE 10 2012 220 578 A1 beschrieben, weiter Endoskope mit einer schwenkbaren Optik bekannt, welche sowohl in prograde als auch in seitliche Richtung blicken können. Solche Endoskope weisen aber keinen Arbeitskanal auf (werden also rein diagnostisch verwendet) oder weisen einen festen Arbeitskanal in prograder Richtung auf und sind somit nicht für die typischen Einsatzzwecke von Duodenoskopen geeignet, die einen seitwärts ausgerichteten Arbeitskanal voraussetzen.

Aus der Offenlegungsschrift DE 10 2010 034 380 A1 ist ein Endoskop bekannt, das über eine Schwenkmechanik in der Art eines Viergelenkgetriebes verfügt, über welche die distale Spitze des Endoskops mit dem proximalen Schaft verbunden werden kann.

Die US 2009/231 419 A1 beschreibt eine Endoskopanordnung mit einem Endoskop, das einen röhrenförmigen Körper, eine Bildgebungsvorrichtung mit retograder Betrachtung, die an einem distalen Endbereich des röhrenförmigen Körpers angeordnet ist, und einen Kanal umfasst, der sich durch den röhrenförmigen Körper erstreckt, und ein Instrument, das sich durch den Kanal des Endoskops erstreckt und aus einer distalen Öffnung des Kanals austritt.

Ferner ist aus US 2012/041264 A1 ein endoskopisches Instrument bekannt, mit einem Gelenkabschnitt eines Schaftes zum biegsamen Verbinden eines distalen Schaftteils mit einem proximalen Teil des Schaftes. Zu diesem Zweck ist der distale Schaftteil mit dem proximalen Schaftteil durch zwei starre Gelenkstangen unterschiedlicher Länge verbunden, die jeweils mit einem Gelenk am distalen und am proximalen Schaftteil ansetzen.

Zusammengefasst sind die dem Stand der Technik bislang bekannten Duodenoskope also sehr teure und aufwendig herzustellende Geräte, die nur für einen sehr spezifischen Einsatzzweck geeignet sind und zudem ein erhebliches Hygienerisiko darstellen.

Angesichts der oben beschriebenen Nachteile des Stands der Technik ist es ein Ziel der vorliegenden Erfindung, ein Endoskop bereitzustellen oder nachzurüsten, welches sowohl als progrades Endoskop als auch als seitwärts ausgerichtetes Endoskop einsetzbar ist/wird. Ein weiteres bevorzugtes Ziel der Erfindung ist es, zur Umgehung der Hygieneproblematik ein solches Endoskop als Einweg-Artikel bereitzustellen oder einen entsprechenden Nachrüstsatz bereitzustellen, welcher als Einweg-Produkt verwendbar ist.

Diese Aufgabe ist durch einen Endoskopkopf mit den Merkmalen des Anspruchs 1 gelöst. Vorteilhafte Weiterbildungen sind Gegenstand der Unteransprüche.

### Kurzbeschreibung der Erfindung

Grundsätzlich weist ein erfindungsgemäßer Endoskopkopf (integrale Lösung) oder ein auf den Endoskopf eines konventionellen Endoskops aufsetzbarer erfindungemäßer (Kappen-) Aufsatz / Endoskopkopf der Endoskop-adaptiven Bauart (adaptive Lösung) zumindest eine Optik zur Bildübertragung, ein Leuchtmittel und einen Arbeitskanal zur Führung von Werkzeugen und/oder zur Durchströmung von Medien auf. Zudem weist der erfindungsgemäße Endoskopkopf/Endoskopkopf-Aufsatz eine interne, zum allgemein bekannten "Deflecting" unterschiedliche (d.h. in Richtung distal davon beabstandete zusätzliche) Schwenkeinrichtung auf, die dazu ausgebildet ist, einen Abschnitt des Endoskopkopfs/Endoskopkopf-Aufsatzes von einer prograden Ausrichtung in eine laterale bzw. seitwärts gerichtete Ausrichtung, d.h. um eine Schwenkachse quer zur Endoskoplängsrichtung, insbesondere stufenlos ggf. aber auch gerastert, zu verschwenken. Zumindest die Optik und der Arbeitskanal sind direkt oder mittelbar mit der Schwenkeinrichtung gekoppelt, derart, dass sie gemeinsam mit der Schwenkeinrichtung bzw. dem von dieser verschwenkbaren Endoskopkopfabschnitt/Aufsatzabschnitt verschwenkt werden. In anderen Worten bildet ein erfindungsgemäßer Endoskopkopf/ Endoskopkopf-Aufsatz eine verschwenkbare Optik- und Arbeitskanaleinheit aus, sodass ein mit diesem bestücktes/nachgerüstetes Endoskop sowohl prograd als auch seitwärts ausgerichtet einsetzbar ist. Das Wort "Optik" steht im Rahmen dieser Anmeldung zusammenfassend für alle dem Stand der Technik im Bereich der Endoskopie bekannte Bildgebungseinrichtungen, wie bspw. einem Modul aus CMOS- oder CCD-Chip und Objektiv im Endoskopkopf/ Endoskopkopf-Aufsatz oder die Verwendung von Lichtleitern etc. Dabei ist es unerheblich, ob die Grundstellung der Schwenkeinrichtung prograd oder lateral ist. Zur Umsetzung des Erfindungsgedankens kommt es lediglich darauf an, dass die Schwenkeinrichtung beide Ausrichtungen einnehmen kann, sodass ein Endoskop mit einem erfindungsgemäßen Endoskopkopf/ Endoskopkopf-Aufsatz die Funktionen eines prograden und eines Endoskops mit Seitoptik in sich vereint. Als Schwenkeinrichtung kommt dabei grundsätzlich jedes dem Stand der Technik bekannte schwenk- oder drehbare Stellglied/Getriebe in Frage. Die schwenkbare Lagerung der Schwenkeinrichtung kann bspw. als Scharnier mit Scharnierbolzen und Buchsen, als Filmscharnier oder als Nase/Kante, die mit einer entsprechenden Führungskontur wechselwirkt, ausgebildet sein. Zum Auslösen einer Verschwenkung der Schwenkeinrichtung kann bspw. ein Bowdenzug verwendet werden, der proximal und außerhalb des Patienten betätigt werden kann. Gemäß einer bevorzugten Ausführungsform kann die Schwenkeinrichtung in einer definierten Position vorgespannt sein.

Ferner weist der Endoskopkopf/ Endoskopkopf-Aufsatz eine Abknickverhinderungsvorrichtung auf, die dazu ausgebildet ist, beim Schwenken der Schwenkeinrichtung in die seitliche Ausrichtung den sich dabei krümmenden Arbeitskanal abzustützen, um ein Abknicken desselben noch wirksamer zu verhindern. Die Abknickverhinderungsvorrichtung stützt den Arbeitskanal in seinem gekrümmten Zustand durch eine gewünschte Formgebung ab. Dies dient dazu, die Passierbarkeit des Arbeitskanals für chirurgische Werkzeuge und Medien sicherzustellen. Vorzugsweise kann die Abknickverhinderungsvorrichtung dazu ausgebildet sein, beim Schwenken der Schwenkeinrichtung in die laterale Ausrichtung auszuklappen/auszufahren und beim Zurückschwenken wieder einzuklappen/einzufahren. Die Ein- und Ausklappkinematik kann bspw. über ein Führungskonturenpaar oder eine Federvorspannung ausgeführt werden.

Vorzugsweise kann neben der Optik und dem Arbeitskanal zusätzlich ein sogenannter Hilfs- oder Nebenkanal zum zusätzlichen Absaugen und Spülen und/oder zumindest ein Leuchtmittel zum gemeinsamen Verschwenken mit der Schwenkeinrichtung gekoppelt sein. In anderen Worten können bei einem bevorzugten Ausführungsbeispiel alle im Stand der Technik üblichen Funktionseinheiten unter Beibehaltung ihrer relativen Ausrichtung zueinander von einer prograden Ausrichtung in eine seitliche Ausrichtung schwenkbar sein.

Eine erfindungsgemäße Schwenkeinrichtung zum Verschwenken von Optik und Arbeitskanal bietet zahlreiche Vorteile gegenüber den im Stand der Technik bekannten Endoskopen. Ein offensichtlicher Vorteil ist, dass ein einziges Gerät nun flexibel für alle Aufgaben eingesetzt werden kann, für welche zuvor die Anschaffung mehrerer Geräte (Gastroskop, Koloskop, Duodenoskop) erforderlich war. Gegenüber handelsüblichen Seitblick-Endoskopen hat ein Endoskop mit erfindungsgemäßem Endoskopkopf/ Endoskopkopf-Aufsatz zudem den Vorteil, dass in der prograden Stellung der Optik die Navigation im und zum Duodenum erheblich vereinfacht wird. D.h., wo zuvor die Endoskopspitze um 90° abgewinkelt werden musste, um mit einem Seitoptik-Endoskopkopf gerade aus blicken zu können, was aufgrund des geringen zur Verfügung stehenden Raumes nur innerhalb des Magens möglich ist, kann das Endoskop mit erfindungsgemäßem Endoskopkopf/ Endoskopkopf-Aufsatz mit prograder Blickrichtung eingeführt und erst am Einsatzort, auf engstem Raum in die seitwärts gerichtete Ausrichtung verschwenkt werden. Ein weiterer Vorteil einer erfindungsgemäßen Optik- und Arbeitskanaleinheit ist ein deutlich vergrößerter möglicher Sichtbereich.

Die Schwenkeinrichtung kann vorzugsweise so ausgebildet sein, dass sich der Durchmesser bzw. die Querschnittsfläche des Endskopkopfs/ Endoskopkopf-Aufsatzes durch das Verschwenken möglichst wenig vergrößert. D.h. konkret, dass sich im Gegensatz zum handelsüblichen Endoskop, das über das Deflecting abgewinkelt werden muss, die Querschnittsfläche des Endoskopkopfs/ Endoskopkopf-Aufsatzes beim Überführen von der prograden in die seitliche Ausrichtung oder andersherum nicht oder kaum vergrößert. In anderen Worten kann die Schwenkeinrichtung dazu ausgebildet sein im Kopf selbst zu Schwenken. Gemäß einer Ausführungsform kann die Schwenkeinrichtung so ausgebildet sein, dass sich die Querschnittsfläche des Endoskopkopfes bei einem solchen Verschwenken um nicht mehr als 25%, bevorzugt nicht mehr als 10%, besonders bevorzugt nicht mehr als 5% vergrößert. Dieses funktionelle Merkmal lässt sich konstruktiv bspw. dadurch umsetzen, dass die Erstreckung des von der Schwenkeinrichtung verschwenkten Endoskopkopfabschnitts/Aufsatzabschnitts in Endoskoplängsrichtung kürzer ist als seine Erstreckung in Endoskopquerrichtung.

Bevorzugt kann die Optik eines erfindungsgemäßen Endoskops einen Blickwinkel (ohne Berücksichtigung von Schwenkbewegungen) zwischen 115° und 140° ermöglichen.

Gemäß einem Aspekt kann die Schwenkeinrichtung im Wesentlichen an einem umfangsseitigen Rand des Endoskopkopfs/ Endoskopkopf-Aufsatzes angeordnet (schwenkbar gelagert) sein. Auf diese Weise kann z.B. ein kompletter distaler Abschnitt des Endoskops/ Endoskopkopf-Aufsatzes, mitsamt aller darauf befindlichen bzw. an diesem angeordneten Funktionseinheiten, verschwenkt werden.

Bei einer solchen Ausführungsform kann der im oder am Endoskopkopf/ Endoskopkopf-Aufsatz ausgebildete Arbeitskanal und/oder der Hilfskanal bevorzugt auf der der Schwenkachse (diametral) gegenüberliegenden Seite angeordnet sein. Dies hat den Vorteil, dass ein möglichst großer Krümmungsradius des Arbeitskanals in der lateralen Stellung erreicht wird, sodass ein Abknicken des Arbeitskanals vermieden werden kann. Gemäß einer vorteilhaften Ausführungsform kann der Arbeitskanal sogar außerhalb und entlang des Endoskopkopfkörpers/ Endoskopkopf-Aufsatzes und im Wesentlichen der Schwenkachse der Schwenkeinrichtung (diametral) gegenüberliegend geführt sein, um so den Abstand zwischen Arbeitskanal und Schwenkachse zur Vermeidung eines Kanalknicks zu vergrößern.

Gemäß einer weiteren Ausführungsform können der Arbeitskanal und/oder der Hilfskanal per se konstruktiv knicksicher ausgebildet sein, z.B. durch Auswahl eines geeigneten Materials (bspw. PTFE) oder das Vorsehen abstützender Schlauchschichten, wie Drahtspiralen.

Gemäß einem weiteren Aspekt der Erfindung, kann der Endoskopkopf/ Endoskopkopf-Aufsatz eine Führungseinrichtung/Visiereinrichtung aufweisen, welche dazu ausgebildet ist, die Ausrichtung der Schwenkeinrichtung anzuzeigen. Dabei ist es von Vorteil, wenn die Ausrichtung der Arbeitskanalöffnung an ein zu untersuchendes Lumen oder sonstiges Gewebe projiziert wird. Zu diesem Zweck kann die Visiereinrichtung bevorzugt ein Lichtzeiger bzw. Laserpointer aufweisen, der im Wesentlichen in Richtung des distalen Ausgangs des Arbeitskanals innerhalb des schwenkbaren Abschnitts des Endoskopkopfs/ Endoskopkopf-Aufsatzes ausgerichtet ist. Dies hat den Vorteil, dass der Anwender während der Behandlung durch die Optik des Endoskops erkennen kann, an welcher Stelle ein durch den abgewinkelten Arbeitskanal ggf. geführtes Werkzeug (medizinisches Instrument) auftreffen wird bzw. wie weit der Anwender die Schwenkeinrichtung abwinkeln muss, um die vatersche Papille zu intubieren.

Gemäß einer bevorzugten Ausführungsform kann der Endoskopkopf/ Endoskopkopf-Aufsatz weiter eine Schlauch- oder Faltenbalgstruktur aufweisen, die die Schwenkeinrichtung mit dem proximal benachbarten nicht verschwenkten Abschnitt des Endoskopkopfs/ Endoskopkopf-Aufsatzes oder Schaftabschnitt verbindet. Wird nun die Schwenkeinrichtung relativ zu dem proximal benachbarten Abschnitts des Endoskopkopfs/ Endoskopkopf-Aufsatzes oder Schaftabschnitts verschwenkt, so wird eine normalerweise durch diese Verschwenkbewegung entstehende Lücke durch den, vorzugsweise in Endoskoplängsrichtung elastischen, Schlauchabschnitt bzw. die Faltenbalgstruktur gegenüber einer Umgebung verschlossen. In anderen Worten kann durch die Schlauch- oder Faltenbalgstruktur der Schwenkraum der Schwenkeinrichtung versiegelt werden. Dabei kann ein solcher Faltenbalg, der sich bei einem Verschwenken des einen Abschnitts des Endoskopkopfs/ Endoskopkopf-Aufsatzes nach Art einer Ziehharmonika längt gleichzeitig die Funktion der vorstehend genannten Abknick-Verhinderungseinrichtung übernehmen, wenn sich der Arbeitskanal radial außerhalb des Endoskopkopfs/ Endoskopkopf-Aufsatzes befindet und sich somit an der Außenseite des Faltenbalgs anlegt.

Gemäß einem besonderen, bevorzugten Aspekt der Erfindung kann der erfindungsgemäße Endoskopkopf als adaptierbarer Endoskopkopf bzw. Endoskopkopfadapter ausgebildet sein. D.h. das Endoskop kann an der distalen Stirnseite des "Deflecting" eine Art Anschlussmaske aufweisen, an die der Endoskopkopf mit der entsprechenden Fähigkeit gemäß vorstehender Beschreibung angekuppelt werden kann oder der Endoskopkopfadapter kann an eine Endoskopkopffäche, vorzugsweise eine Stirnfläche, eines bereits bekannten Endoskops lösbar befestigt werden und damit als Nachrüstaufsatz gemäß vorstehender Beschreibung dienen. In anderen Worten lässt sich ein handelsüblicher Endoskopkopf mit Hilfe eines solchen Adapters zu einem erfindungsgemäßen Endoskop mit einer Schwenkeinrichtung zum Verschwenken der Optik und des Arbeitskanals umrüsten. Ein solcher Endoskopkopfadapter kann sich die bereits vorhandenen Funktionseinheiten des umzurüstenden Endoskops zu Eigen machen, d.h. er kann betriebswirksam mit der Optik und/oder den Leuchtmitteln und/oder dem Arbeitskanal verbunden/gekoppelt werden und dazu ausgebildet sein, zumindest die adapterinterne Optik und den Arbeitskanal über die Axiallänge des Adapters weiter zu leiten und mit Hilfe der Schwenkeinrichtung entsprechend zu verschwenken. Genauso kann der Endoskopkopfadapter aber auch seine eigene interne Optik und/oder Leuchtmittel und/oder einen eigenen am umgerüsteten Endoskop entlang geführten Arbeitskanal (zusätzlich/parallel zu den im Endoskopkopf verbauten Elementen) aufweisen. Ebenso ist jegliche Kombination möglich, d.h. dass einige Funktionseinheiten des umgerüsteten Endoskops genutzt werden, während andere Funktionseinheiten unabhängig vom umgerüsteten Endoskop (parallel dazu) ausgebildet sind. Die Adaptierbarkeit eines solchen als Adapter ausgeführten Endoskopkopfs lässt sich bspw. durch eine Gewindepaarung, einen Rast- oder Aufsteckmechanismus oder ein Festsaugen mittels Unterdruck ausführen. Ein erfindungsgemäßer Endoskopkopfadapter kann vorzugsweise als Einweg-Produkt konzipiert sein, wohingegen das Endoskop z.B. ein Mehrweg-Produkt sein kann. Dies hat den Vorteil, dass ein handelsübliches progrades Endoskop mit der erfindungsgemäßen Schwenkeinrichtung ausgerüstet werden kann, die hygienekritischsten Komponenten (vor allem die verschiedenen Kanäle) aber nach Gebrauch entsorgt werden können.

Ein weiterer ggf. unabhängig zu beanspruchender Aspekt der Erfindung betrifft ein Endoskop, insbesondere ein Einweg-Endoskop, mit einem oben beschriebenen erfindungsgemäßen Endoskopkopf/ Endoskopkopf-Aufsatz. Um eine Behandlung im Bereich des Duodenums zu ermöglichen, weist ein solches Endoskop, neben einem oben beschriebenen erfindungsgemäßen Endoskopkopf/ Endoskopkopf-Aufsatz, einen vorzugsweise flexiblen Schaft auf, durch den und/oder entlang dessen sich diverse Betriebs- und Versorgungskanäle ziehen, die eine Verbindung der verschiedenen Funktionseinheiten des Endoskopkopfs mit einer proximalen Betriebsstation sowie einer Steuerung/Handhabe ermöglichen. Durch eine Ausführung des erfindungsgemäßen Endoskops als Einweg-Endoskop, das an ein mehrfach verwendbares Griffteil anschließbar ist, erübrigt sich die oben besprochene Hygiene-Thematik, da alle in den Körper eingeführten Komponenten nach der Behandlung entsorgt werden.

Vorzugsweise kann ein erfindungsgemäßes Endoskop einen aktiv abkrümmbaren distalen Schaftabschnitt, ein sogenanntes Deflecting, aufweisen, welcher dazu ausgebildet ist, durch die Steuerung/Handhabe von außerhalb des Patienten vom Anwender betätigt zu werden und so ein Abkrümmen des Deflectings in zumindest eine Richtung zu ermöglichen. Das Deflecting grenzt vorzugsweise proximal an den Endoskopkopf an. Als Deflecting kommen alle im Stand der Technik üblichen Ausführungsformen in Frage, bspw. eine Ausführung mit Bowdenzügen und gelenkig miteinander verbundenen Wirbeln/Gliedern oder eine Ausführung mit hydraulischen Stellgliedern.

Gemäß einer weiter bevorzugten Ausführungsform kann das erfindungsgemäße Endoskop ein Rotationsgetriebe bzw. eine Drehkinematik zum Rotieren eines distalen Abschnitts des Endoskops oder des Endoskopkopfs um dessen Längsachse aufweisen. In anderen Worten ist es von Vorteil, wenn sich zumindest der Abschnitt des Endoskops bzw. des Endoskopkopfes, in welchem die Schwenkeinrichtung angeordnet ist, um die Endoskoplängsachse verdreht werden kann. Auf diese Weise lässt sich der Wirkungsradius der verschwenkbaren Optik- Arbeitskanaleinheit noch einmal deutlich vergrößern. Bevorzugt kann das Drehgetriebe zwischen dem proximalen Ende eines (aktiv abkrümbaren) Deflectings und dem distalen Ende des flexiblen, passiv abkrümmbaren Schafts angeordnet werden. Vorzugsweise kann ein so drehbares erfindungsgemäßes Endoskop ein in eine einzige Richtung abwinkelbares Deflecting aufweisen. Auf diese Weise kann das (rotierbare) Deflecting in praktisch jede Richtung abgewinkelt werden, während das Deflecting einfacher, günstiger und schlanker produziert werden kann.

### Figurenbeschreibung

Die Erfindung wird nachstehend anhand bevorzugter Ausführungsbeispiele unter Bezugnahme auf die begleitenden Figuren näher erläutert.

Es zeigen
Fig. 1 eine Skizze eines prograden Endoskops gemäß dem Stand der Technik bei der Exploration eines Duodenums;
Fig. 2 eine Detaildarstellung eines dem Stand der Technik bekannten Duodenoskops bei einem Eingriff an der vaterschen Papille;
Fig. 3 eine Darstellung eines ersten Ausführungsbeispiels für ein erfindungsgemäßes Endoskop in einer unausgelenkten prograden Ausrichtung;
Fig. 4 eine Darstellung des ersten Ausführungsbeispiels für ein erfindungsgemäßes Endoskop in einer ausgelenkten lateralen bzw. seitwärts blickenden Ausrichtung;
Fig. 5 eine Darstellung eines zweiten Ausführungsbeispiels für ein erfindungsgemäßes Endoskop in einer ausgelenkten lateralen bzw. seitwärts blickenden Ausrichtung; und
Fig. 6 ein Endoskopkopfadapter gemäß einer bevorzugten Ausführungsform der Erfindung.

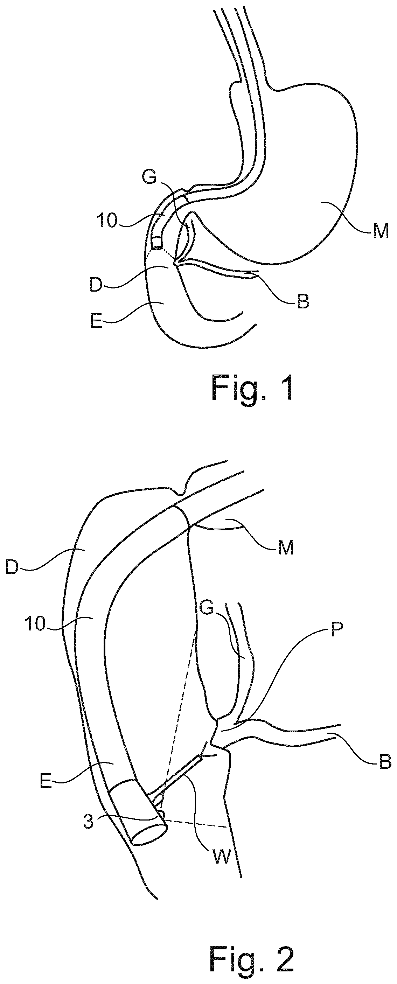

Die Fign. 1 und 2 dienen der Erläuterung eines bevorzugten Einsatzgebietes eines erfindungsgemäßen Endoskops bzw. eines erfindungsgemäßen Endoskopkopfs sowie der bislang dem Stand der Technik zu diesem Zweck bekannten Endoskope.

Wie aus Fig. 1 ersichtlich ist, ist die Papilla Vateri (P) (der Eintritt des Gallengangs (G) und des Bauchspeicheldrüsengangs (B) in den Zwölfingerdarm) im rückwärtigen (dorsalen) absteigenden Teil (Pars descendens) des Zwölffingerdarms (Duodenum) (D) lokalisiert und durch die gewundene Geometrie dieses Systems relativ schwer zugänglich. Der zur Verfügung stehende Raum im Bereich des Duodenums (D) ist sehr begrenzt, wodurch Eingriffe an der Papilla vateri (P) mit prograden Endoskopen nicht möglich sind, da bei einem entsprechenden Abwinkeln der Endoskopspitze bzw. des Deflectingabschnitts 10 in Richtung Eingriffsabschnitt nicht mehr genügend Distanz zum Lumen des Duodenums (D) für eine ordentliche Bildgebung bliebe. Ein Abkrümmen des Deflectings 10 im Duodenum (D) stellt aufgrund des engen Raumes zudem ein Verletzungsrisiko dar. Schlimmstenfalls könnte gar das Lumen des Duodenums bei einem solchen Manöver reißen.

Aus diesem Grund sind dem Stand der Technik die eingangs genannten Duodenoskope (E) bekannt, die eine seitwärts blickende Optik sowie einen entsprechend ausgerichteten Arbeitskanal aufweisen, um den zur Verfügung stehenden Raum optimal zu nutzen (vgl. Fig. 2). Da eine solche Optik einen Blickwinkel von 125° bis 140° besitzt, kann das Endoskop auch rückblickend/retrograd verwendet werden. Solche Duodenoskope (E) haben jedoch den Nachteil, dass sie in ihrer lateralen Ausrichtung der Optik und des Arbeitskanals festgelegt sind. Dies erschwert zum einen die allgemeine Navigation innerhalb des Patienten und macht solche Endoskope zum anderen unflexibel in ihren Einsatzmöglichkeiten. Zudem sind solche Duodenoskope bislang nicht sterilisierbar sondern lediglich desinfizierbar, wodurch sie ein erhebliches Hygienerisiko darstellen.

Ein Grundgedanke der vorliegenden Erfindung ist es deshalb, ein Endoskop, insbesondere einen Endoskopkopf oder einen Endoskopkopf-Aufsatz bereitzustellen, bei dem interne Optik und Arbeitskanal dazu ausgebildet sind, gemeinsam um mindestens 90° verschwenkbar zu sein (ohne dass das Deflecting aktiv geschwenkt werden muss), um einen Betrieb sowohl in prograder Ausrichtung als auch in lateraler Ausrichtung zu ermöglichen. Anders ausgedrückt, ist es das Hauptanliegen der vorliegenden Erfindung ein Kombi-Gerät bereitzustellen, dass die Funktionen und Vorteile eines prograden Gastro- oder Koloskops mit denen eines Duodenoskops in einem Gerät vereint.

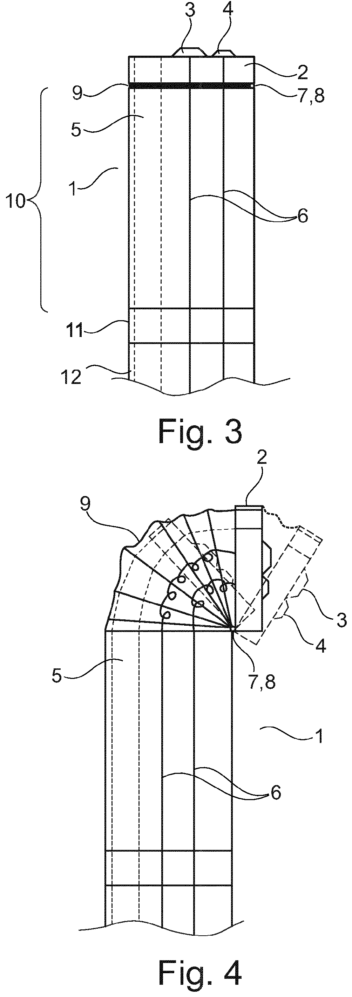

In Fig. 3 ist eine erste beispielhafte Ausführungsform eines erfindungsgemäßen Endoskops 1 in einer ersten prograden Ausrichtung dargestellt. Das Endoskop 1 weist an seinem distalen Ende einen Endoskopkopf 2 auf, der verschiedene Funktionseinheiten wie eine Optik 3; ein Leuchtmittel 4 und einen Arbeitskanal 5 aufweist. Der Übersichtlichkeit halber sind hier und im Folgenden nur die oben genannten nötigsten Funktionseinheiten dargestellt, selbstverständlich kann ein erfindungsgemäßer Endoskopkopf zusätzlich diverse andere dem Stand der Technik bekannte Funktionseinheiten, etwa Reinigungsdüsen für ein Objektiv der Optik 3 etc. aufweisen.

Der Arbeitskanal 5 erstreckt sich von seiner distalen Öffnung bzw. seinem Ausgang an der distalen Stirnseite des Endoskopkopfs 2, durch einen flexiblen Schaft bzw. entlang eines flexiblen Schaftes eines mit dem Endoskopkopf 2 bestückten Endoskops 1, bis hin zu einer proximalen Öffnung im Bereich des Endoskopgriffs und kann so bspw. zum Einführen von chirurgischen Instrumenten oder Werkzeugen (W), wie einem Papillotom, oder zum Applizieren von Medien im Patienten verwendet werden. Das dargestellte Endoskop 1 weist weiter Funktions- und Versorgungskanäle 6, wie bspw. elektrische Leitungen, zum Versorgen der Funktionseinheiten, zur Übermittlung von Daten und zum Steuern der Bewegungen des Endoskops auf, die in proximaler Richtung an eine Betriebsstation (nicht dargestellt) bzw. einen Controller/ ein Steuergerät (ebenfalls nicht dargestellt) anschließbar sind. Darüber hinaus kann ein solches Endoskop der Schaftbauart zwischen dem Endoskopkopf und dem vorzugsweise flexiblen Endoskopschaft ein sogenanntes Deflecting aufweise, das in Verlängerung des passiv biegsamen Endoskopschafts einen aktiv abkrümmbaren Schaftabschnitt darstellt. Dieser aktiv abkrümmbare Schaftabschnitt kann entweder in alle Richtungen abkrümbar sein oder ggf. nur in eine Richtung, wobei im letzteren Fall zwischen Deflecting und Endoskopschaft ein Rotoationskranz angeordnet sein kann, der das Deflecting bezüglich des Endoskopschafts um die Endoskoplängsachse drehbar hält.

Um ein Überführen des Endoskopkopfs 2 bzw. eines vorzugsweise distalen Kopfabschnitts von einer prograden Ausrichtung in eine seitwärts blickende Ausrichtung zu ermöglichen, weist das Endoskop bzw. der Endoskopkopf eine Schwenkvorrichtung 7 auf. Diese ist im ersten Ausführungsbeispiel als eine Kombination aus einem Scharnier 8 und einem (nicht dargestellten) Bowdenzug umgesetzt und ermöglicht im gezeigten Beispiel ein Verschwenken des gesamten Endoskopkopfs 2 vorzugsweise um einen Winkel > 90°, sodass die Stirnfläche des Endoskopkopfs 2 nach dem Verschwenken lateral, also seitlich bis rückblickend, ausgerichtet ist. Das Scharnier 8 kann beispielsweise durch einen in Buchsen geführter Bolzen, ein Filmscharnier oder eine Nase, die in einer entsprechenden Nut/Führungskontur geführt ist, umgesetzt sein.

Da sich bei einem solchen Verschwenkvorgang eine Öffnung oder Spalt zwischen dem verschwenkten Kopf 2 und dem proximal an diesem angrenzenden Schaftabschnitt auftut, ist zur Aufrechterhaltung der Sterilbarriere im Bereich des Schwenkraums als Abdichtelement 9 z.B. ein Faltenbalg vorgesehen, der in Endoskoplängsrichtung, insbesondere auf der dem Scharnier abgewandten Seite, aufweitbar/dehnbar ist. Anstelle eines Faltenbalgs kann auch z.B. ein elastisches schlauchförmiges Verbindungselement verwendet werden. Wichtig ist nur, dass das Abdichtelement 9 zusammen mit dem Kopf und dem Schaft ein versiegeltes Lumen bildet und in Längsrichtung reversibel aufgeweitet/gedehnt werden kann, um die durch das Verschwenken zurückgelegte Distanz zu überbrücken. Ebenfalls zum Zwecke einer reversiblen Verlängerbarkeit sind die Funktions- und Versorgungskanäle 6 im gezeigten ersten bevorzugten Ausführungsbeispiel spiralförmig aufgewickelt. Auch der Arbeitskanal 5 ist im Schwenkbereich vorzugsweise als Faltenbalg oder elastischer Schlauch ausgeführt.

Proximal an das Abdichtelement 9 grenzt im gezeigten Ausführungsbeispiel ein abkrümmbarer Schaftabschnitt 10, das sogenannte Deflecting, an, wie dieses vorstehend bereits angedeutet wurde. Dieses kann bspw., wie im Stand der Technik üblich, eine Vielzahl hintereinandergeschalteter und verschwenkbar miteinander verbundener Glieder/Wirbel aufweisen, die z.B. mit Hilfe von Bowdenzügen oder hydraulischen Stellgliedern in eine oder mehrere Richtungen abgekrümmt werden können. Insbesondere kann durch ein solches Deflecting 10 ein Abkrümmungsgrad von mindestens 180° in eine Richtung erreicht werden.

Proximal an das Deflecting grenzt in der ersten bevorzugten Ausführungsform ein Rotationsgetriebe 11 an, welches somit zwischen das Deflecting 10 und dem passiv flexiblen Endoskopschaft 12 geschaltet ist. Das Rotationsgetriebe 11 ist dazu ausgebildet, eine Relativdrehung zwischen den proximal und distal an demselben angrenzenden Schaftabschnitten 10, 12 zu ermöglichen. Dies hat den Vorteil, dass zum Ausrichten des Endoskopkopfs 2, insbesondere im verschwenkten Zustand, nicht das gesamte Endoskop 1 gedreht werden muss, was erhebliche Unannehmlichkeiten für den Patienten zur Folge hätte. Stattdessen lässt sich auf diese Weise gezielt ein distaler Abschnitt des Endoskops in eine gewünschte Ausrichtung rotieren. Ebenso gut könnte das Rotationsgetriebe direkt proximal an den Endoskopkopf 2 angrenzen. Das Rotationsgetriebe 11 kann durch diverse dem Stand der Technik bekannte Getriebe oder Drehdurchführungen zur konzentrischen Relativdrehung zweier Komponenten umgesetzt werden. Typische Beispiele sind Drehkränze/Drehwerke, Planetengetriebe oder elektro-hydraulische Schleifringsysteme.

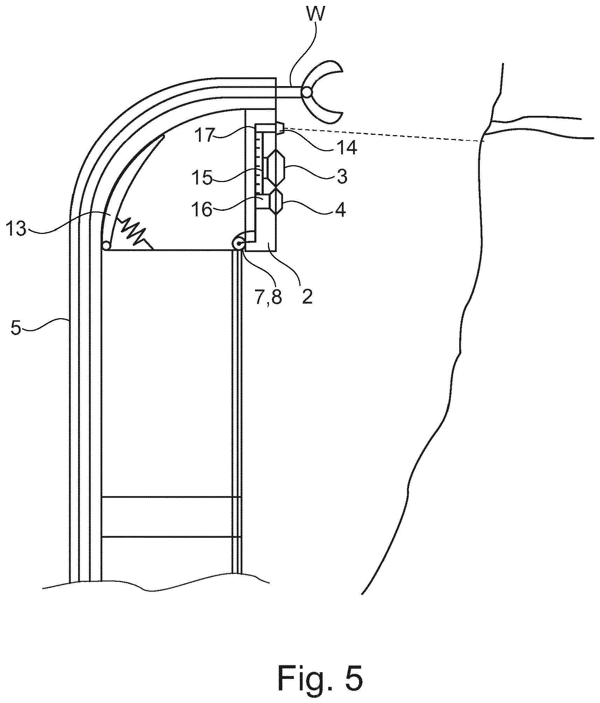

In Fig. 5 ist ein Endoskop mit einem Endoskopkopf gemäß einem zweiten bevorzugten Ausführungsbeispiel dargestellt. Wie hier beispielhaft dargestellt, kann der Arbeitskanal 5 auch außen am Endoskopschaft- und Kopf geführt sein, vorzugsweise an der dem Scharnier 8 diametral gegenüberliegenden bzw. abgewandten Seite, um den Biegeradius des Arbeitskanals 5 in der lateralen/seitwärts gerichteten Ausrichtung zu maximieren. Als zusätzliches Merkmal ist bei dieser Ausführungsvariante optional eine Abknickverhinderungsvorrichtung 13 bzw. ein Stützelement angeordnet, welches eine Formkontur vorgibt, entlang derer sich der Arbeitskanal 5 krümmt. Auf diese Weise kann ein Abknicken des Arbeitskanals 5 und ein daraus resultierendes Blockieren des Durchgangs für medizinische Instrumente/Werkzeuge unterbunden werden. Die Abknickverhinderungsvorrichtung 13 ist vorzugsweise dazu ausgebildet, beim Schwenken der Schwenkeinrichtung 7 in die laterale Ausrichtung aufzuklappen, bspw. durch eine Federvorspannung, und beim Zurückschwenken der Schwenkeinrichtung 7 wieder einzuklappen, bspw. durch eine Führungskontur. Alternativ kann aber auch der Faltenbalg gemäß vorstehender Beschreibung dazu genutzt werden, den außerhalb des Faltenbalgs geführten Arbeitskanal radial abzustützen und so ein Abknicken zu vermeiden.

Bei der gezeigten zweiten Ausführungsform ist das Abdichtelement 9 als elastischer Silikon-Schlauch ausgeführt. In diesem Ausführungsbeispiel werden die elektrischen Funktions- und Versorgungskanäle im Randbereich des Schafts und über das Scharnier 8 in den Endoskopkopf geführt, um dort die auf einer Platine 17 angeordneten als LEDs 16 ausgeführten Leuchtmittel 4 und einen als CMOS-Chip ausgeführten Bildsensor 15 der Optik-Einheit 3 zu versorgen. Im Endoskopkopf 2 bzw. auf der Platine 17 ist im gezeigten Ausführungsbeispiel zudem ein Lichtzeiger 14 bzw. eine Laser-Visiereinrichtung angeordnet, die im Wesentlichen parallel zum distalen Ausgang des Arbeitskanals 5 angeordnet ist, um den ungefähren Auftrittspunkt eines durch den Arbeitskanal geführten Werkzeugs an einer Zielstelle im Patienten durch einen Lichtpunkt zu markieren/anzuzeigen.

Die Optik 3 ist im gezeigten Beispiel als CMOS-Chip 15 der mit einem Linsensystem kooperiert ausgeführt. Ebenso sind jedoch andere im Stand der Technik bekannte Lösungen denkbar. So kann der Bildsensor 15 bspw. auch in proximale Richtung verlagert sein und das Bild mittels Lichtleitern (Prismen und/oder Glasfasern) zu diesem geleitet werden. Ebenso kann das bzw. können die Leuchtmittel 4 in proximale Richtung verlagert sein.

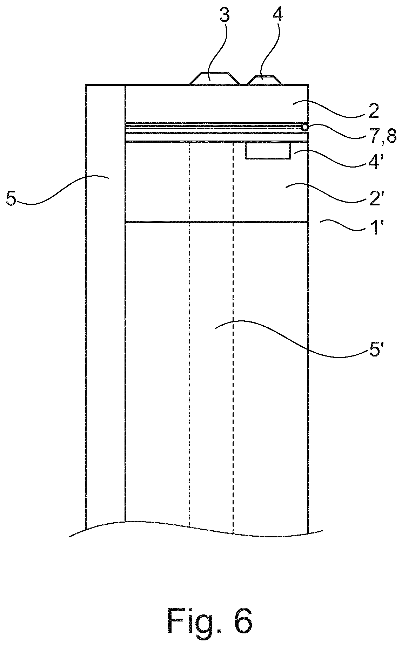

Fig. 6 zeigt eine weitere, ggf. unabhängig zu beanspruchende Ausführungsform der vorliegenden Erfindung. Der dargestellte Endoskopkopf 2 ist in diesem Fall als separater Adapter (Endoskopkopf-Aufsatz) ausgeführt, der lösbar an/auf einem handelsüblichen Endoskop 1' bzw. dessen internem Endoskopkopf 2' befestigbar ist. Der hier zentral angeordnete Arbeitskanal 5' des umgerüsteten Endoskops 1' kann ferner genutzt werden, um einen entsprechend vorbereiteten Abschnitt der Unterseite des Endoskopkopfadapters 2 mit einem Unterdruck zu beaufschlagen und somit den Endoskopkopfadapter 2 am Endoskop 1' zu fixieren. Die Optik 3 des dargestellten Endoskopkopfadapters 2 ist unabhängig von der nicht dargestellten internen Optik des Endoskops 1'. Zudem weist der dargestellte Endoskopkopfadapter 2 einen eigenen Arbeitskanal 5 zur Führung von Werkzeugen sowie einen eigenen Hilfskanal auf, die entlang des Endoskops 1' (außenseitig) angeordnet und dazu ausgebildet sind, gemeinsam mit der Schwenkeinrichtung 7 zu verschwenken. Weiter weist der dargestellte Endoskopkopfadapter 2 eigene Leuchtmittel 4 auf. Der so ausgebildete autarke Endoskopkopfadapter 2 ist weiter an eine eigene Steuerungseinrichtung (separat zu der des Endoskops 1') zum Steuern der Schwenkeinrichtung sowie an eigene Stromversorgungs- und Pumpenanschlüsse angeschlossen oder anschließbar.

Entscheidend bei sämtlichen, vorstehend genannten Ausführungsbeispielen, d.h. bei der integralen oder der adaptiven Lösung ist es, dass der Arbeitskanal, sowie die für die Bildgebung aktuell verantwortliche Optik gleichzeitig um den gleichen Winkel verschwenkt werden, sodass die Optik und die distale Öffnung des Arbeitskanals immer in die gleiche, aufeinander abgestimmte Richtung ausgerichtet sind. Dadurch wird gewährleistet, dass das Endoskop für unterschiedliche Zwecke optimal einsetzbar ist. Insbesondere dann, wenn das adaptive Lösungskonzept praktisch umgesetzt wird, erzielt man den zusätzlichen Vorteil, dass handelsübliche Endoskope verwendbar sind, wobei das Hygieneproblem auf elegante Weise dadurch gelöst werden kann, indem der adaptive Endoskopkopf-Aufsatz mit schwenkbarem, eine zusätzliche/separate Optik und zusätzlichen/separaten Arbeitskanal aufweisenden Aufsatzabschnitt als Einweg-Produkt vorgesehen wird.

### Bezugszeichen

- 1: Endoskop;
- 2: Endoskopkopf;
- 3: Optik;
- 4: Leuchtmittel;
- 5: Arbeitskanal;
- 6: Funktion- und Versorgungskanäle;
- 7: Schwenkeinrichtung;
- 8: Schwenkachse/Scharnier;
- 9: Abdichtelement/Faltenbalg;
- 10: Deflecting;
- 11: Rotationsgetriebe;
- 12: flexibler Schaft;
- 13: Abknickverhinderungsvorrichtung;
- 14: Lichtzeiger;
- 15: Bildsensor/CMOS;
- 16: LED;
- 17: Platine;
- B: Bauchspeicheldrüsengang;
- D: Duodenum;
- E: Endoskop (Duodenoskop) gemäß dem Stand der Technik;
- G: Gallengang;
- M: Magen;
- P: Papilla vateri; und
- W: Werkzeug.

## Patentansprüche

1. Endoskopkopf (2) der Endoskop-integralen Bauart oder der Endoskop-adaptiven Bauart nach Art eines zusätzlichen Endoskopkopf-Aufsatzes jeweils mit zumindest
- einer eigenen Optik (3) zur Bildübertragung;
- einem eigenen Leuchtmittel (4) oder Lichtleiter; und
- einem eigenen Arbeitskanal (5) zur Führung von medizinischen Werkzeugen (W) und/oder zur Durchströmung von Medien, wobei
der Endoskopkopf (2) der integralen oder adaptiven, aufsatzartigen Bauart eine interne Schwenkeinrichtung (7) aufweist, die dazu ausgebildet ist zumindest einen Abschnitt des Endoskopkopfs (2) von einer prograden Ausrichtung in eine laterale oder eine retrograde Ausrichtung, insbesondere stufenlos, zu verschwenken und
zumindest die Optik (3) und der Arbeitskanal (5) direkt oder mittelbar mit der Schwenkeinrichtung (7) gekoppelt sind, derart, dass sie gemeinsam mit dieser unter Beibehaltung ihrer relativen Ausrichtung zueinander verschwenkbar sind,
**gekennzeichnet durch**
eine Abknickverhinderungsvorrichtung (13), die dazu ausgebildet ist, beim Schwenken der Schwenkeinrichtung (7) in die laterale Ausrichtung den sich dabei krümmenden Arbeitskanal (5) durch eine gewünschte Formgebung abzustützen .

2. Endoskopkopf (2) gemäß Anspruch 1, **dadurch gekennzeichnet, dass** eine Schwenkachse (8) der Schwenkeinrichtung (7) im Wesentlichen im Randbereich einer Querschnittsfläche des Endoskopkopfes (2) angeordnet ist.

3. Endoskopkopf (2) gemäß Anspruch 2, **dadurch gekennzeichnet, dass** der Arbeitskanal (5) im oder am Endoskopkopf auf einer der Schwenkachse (8) der Schwenkeinrichtung (7) gegenüberliegenden Seite der Endoskop-Querschnittsfläche angeordnet ist.

4. Endoskopkopf (2) gemäß Anspruch 2 oder 3, **dadurch gekennzeichnet, dass** die Schwenkeinrichtung (7) eine schwenkbare Lagerung in Form eines Scharniers (8) aufweist, wobei die elektrischen Funktions- und Versorgungskanäle der Optik (3) und der Leuchtmittel (4) über das Scharnier (8) in den Endoskopkopf (2) geführt sind.

5. Endoskopkopf (2) gemäß einem der vorgenannten Ansprüche,
**gekennzeichnet durch** eine Führungseinrichtung, insbesondere einen Lichtzeiger (14), welche dazu ausgebildet ist, die Ausrichtung der Schwenkeinrichtung und/oder des distalen Arbeitskanalausgangs relativ zu einem benachbarten Endoskopkopf- oder Schaftabschnitt durch Projizieren der Ausrichtung an ein zu untersuchendes Lumen oder sonstiges Gewebe anzuzeigen.

6. Endoskopkopf (2) gemäß einem der vorgenannten Ansprüche, **gekennzeichnet durch** ein, insbesondere schlauchförmiges oder faltenbalgförmiges, in Endoskoplängsrichtung reversibel verlängerbares Abdichtelement (9), das die Schwenkeinrichtung (7) mit einem proximal benachbarten Endoskopkopf- oder Schaftabschnitt verbindet, zu welchem die Schwenkeinrichtung (7) relativ verschwenkbar ist, und einen Schwenkraum der Schwenkeinrichtung (7) in der lateralen oder retrograden Ausrichtung derselben, gegenüber einer Umgebung versiegelt.

7. Endoskopkopf (2) gemäß einem der vorgenannten Ansprüche, **dadurch gekennzeichnet, dass** die Abknickverhinderungsvorrichtung (13)dazu ausgebildet ist, in der lateralen oder retrograden Ausrichtung der Schwenkeinrichtung (7) aufzuklappen, um den mitgeführten Arbeitskanal (5) von radial innenseitiger Richtung her durch die Vorgabe einer Formkontur, entlang derer sich der Arbeitskanal (5) krümmt, gegen ein Abknicken abzustützen.

8. Endoskopkopf (2) gemäß einem der Ansprüche 1 bis 7, **dadurch gekennzeichnet, dass** dieser dazu ausgebildet ist als Endoskopkopfadapter lösbar an einer Endoskopkopffläche oder einem Endoskopschaft eines Endoskops befestigt und mit einer Optik und/oder einem Leuchtmittel und/oder einem Arbeitskanal des Endoskops betriebswirksam gekoppelt zu werden.

9. Endoskop (1) mit einem flexiblen Schaft (12), der insbesondere einen Deflectingabschnitt (10) aufweist, und einer Anzahl an Funktions- und Versorgungskanälen (6), die an eine Betriebsstation anschließbar sind, **gekennzeichnet durch** einen Endoskopkopf (2) der integralen oder adaptiven, aufsatzartigen Bauart gemäß einem der Ansprüche 1 bis 8.

10. Endoskop (1) gemäß Anspruch 9, **gekennzeichnet durch** zumindest ein Rotationsgetriebe (11) zum Rotieren eines distalen Abschnitts des Endoskops (1) oder des Endoskopkopfs (2) um dessen Längsachse.

## Claims

1. Endoscope head (2) of the endoscope-integral type or of the endoscope-adaptive type in the form of an additional endoscope-head attachment each with at least
- an optical unit (3) of its own for image transfer;
- a lighting means (4) or a light guide of its own; and
- a working channel (5) of its own for the guidance of medical tools (W) and/or throughflow of media, wherein
the endoscope head (2) of the integral or adaptive, attachment-like type has an internal pivoting device (7) which is designed to pivot, in particular continuously, at least a portion of the endoscope head (2) from a prograde orientation into a lateral or a retrograde orientation, and
at least the optical unit (3) and the working channel (5) are coupled directly or indirectly to the pivoting device (7) such that they are pivotable together with the latter while maintaining their relative orientation with respect to each other,
**characterized in that**
a kinking prevention device (13) which is configured to support the working channel (5), which bends during pivoting of the pivoting device (7) into the lateral orientation, via a desired shape.

2. Endoscope head (2) according to claim 1, **characterized in that** a pivot axis (8) of the pivoting device (7) is arranged substantially in the edge region of a cross-sectional area of the endoscope head (2).

3. Endoscope head (2) according to claim 2, **characterized in that** the working channel (5) is arranged in or on the endoscope head on a side of the endoscope cross-section opposite the pivot axis (8) of the pivoting device (7).

4. Endoscope head (2) according to claim 2 or 3, **characterized in that** the pivoting device (7) has a pivotable bearing in the form of a hinge (8), wherein the electrical functional and supply channels of the optical unit (3) and of the lighting means (4) are guided via the hinge (8) into the endoscope head (2).

5. Endoscope head (2) according to one of the preceding claims,
**characterized by** a guiding device, in particular a light pointer (14), which is designed to indicate the orientation of the pivoting device and/or of the distal working channel exit relative to an adjacent endoscope head or shaft portion by projecting the orientation onto a lumen or other tissue to be examined.

6. Endoscope head (2) according to one of the preceding claims, **characterized by** a sealing element (9), which has in particular a tubular or bellows-like shape, which can be reversibly extended in the longitudinal direction of the endoscope and which connects the pivoting device (7) to a proximally adjacent endoscope head or shaft portion, to which the pivoting device (7) can be relatively pivoted, and which seals a pivoting space of the pivoting device (7) in the lateral or retrograde orientation thereof with respect to an environment.

7. Endoscope head (2) according to one of the preceding claims, **characterized in that** the kinking prevention device (13) is designed to fold open in the lateral or retrograde orientation of the pivoting device (7) in order to support the entrained working channel (5) from the radially inward direction against kinking by specifying a contour shape along which the working channel (5) curves.

8. Endoscope head (2) according to one of claims 1 to 7, **characterized in that** it is designed to be detachably fastened as an endoscope head adapter to an endoscope head surface or an endoscope shaft of an endoscope and to be operatively coupled to an optical unit and/or a lighting means and/or a working channel of the endoscope.

9. Endoscope (1) having a flexible shaft (12), which in particular has a deflecting portion (10), and a number of functional and supply channels (6), which can be connected to an operating station, **characterized by** an endoscope head (2) of the integral or adaptive, attachment-like type in accordance with one of claims 1 to 6.

10. Endoscope (1) according to claim 9, **characterized by** at least one rotary transmission (11) for rotating a distal portion of the endoscope (1) or of the endoscope head (2) about its longitudinal axis.

## Revendications

1. Tête d'endoscope (2) du type intégré à l'endoscope ou du type s'adaptant à l'endoscope comme un élément rapporté de tête d'endoscope supplémentaire respectivement avec au moins
- une optique (3) propre pour la transmission d'images ;
- un moyen d'éclairage (4) ou guide de lumière propre ; et
un canal de travail (5) propre pour le guidage d'outils (W) médicaux et/ou l'écoulement de milieux, dans lequel
la tête d'endoscope (2) du type intégré ou adaptatif, de type élément rapporté présente un appareil de pivotement interne (7) qui est configuré afin de pivoter au moins une section de la tête d'endoscope (2) depuis une orientation prograde dans une orientation latérale ou rétrograde, en particulier en continu et
au moins l'optique (3) et le canal de travail (5) sont couplés directement ou indirectement à l'appareil de pivotement (7) de telle manière qu'ils puissent être pivotés conjointement avec celui-ci en conservant leur orientation relative l'un par rapport à l'autre,
**caractérisée par**
un dispositif d'empêchement de rupture (13) qui est configuré afin de soutenir le canal de travail (5) se courbant par un formage souhaité lors du pivotement de l'appareil de pivotement (7) dans l'orientation latérale.

2. Tête d'endoscope (2) selon la revendication 1, **caractérisée en ce qu'**un axe de pivotement (8) de l'appareil de pivotement (7) est agencé sensiblement dans la zone de bord d'une aire de section de la tête d'endoscope (2).

3. Tête d'endoscope (2) selon la revendication 2, **caractérisée en ce que** le canal de travail (5) est agencé dans ou au niveau de la tête d'endoscope sur un côté opposé à l'axe de pivotement (8) de l'appareil de pivotement (7) de l'aire de section d'endoscope.

4. Tête d'endoscope (2) selon la revendication 2 ou 3, **caractérisée en ce que** l'appareil de pivotement (7) présente un logement pivotant sous la forme d'une charnière (8), dans laquelle les canaux de fonction et d'alimentation électriques de l'optique (3) et des moyens d'éclairage (4) sont guidés dans la tête d'endoscope (2) par le biais de la charnière (8).

5. Tête d'endoscope (2) selon l'une quelconque des revendications précédentes, **caractérisée par** un appareil de guidage, en particulier un indicateur lumineux (14), qui est configuré afin d'indiquer l'orientation de l'appareil de pivotement et/ou de la sortie de canal de travail distale par rapport à une section de tête d'endoscope ou de tige contiguë par projection de l'orientation sur un lumen ou un autre tissu à examiner.

6. Tête d'endoscope (2) selon l'une quelconque des revendications précédentes, **caractérisée par** un élément étanche (9) en particulier en forme de tuyau ou de soufflet, prolongeable de manière réversible dans le sens longitudinal de l'endoscope qui relie l'appareil de pivotement (7) à une section de tête d'endoscope ou de tige contiguë de manière proximale, par rapport à laquelle l'appareil de pivotement (7) peut être pivoté relativement, et scelle un espace de pivotement de l'appareil de pivotement (7) dans l'orientation latérale ou rétrograde par rapport à un environnement.

7. Tête d'endoscope (2) selon l'une quelconque des revendications précédentes, **caractérisée en ce que** le dispositif d'empêchement de rupture (13) est configuré afin de se rabattre dans l'orientation latérale ou rétrograde de l'appareil de pivotement (7) afin de soutenir le canal de travail (5) entraîné contre une rupture radialement depuis le sens de côté intérieur par la prescription d'un contour de forme, le long duquel le canal de travail (5) se courbe.

8. Tête d'endoscope (2) selon l'une quelconque des revendications 1 à 7, **caractérisée en ce que** celle-ci est configurée afin d'être fixée comme adaptateur de tête d'endoscope de manière détachable au niveau d'une surface de tête d'endoscope ou d'une tige d'endoscope d'un endoscope et couplée de manière opérationnelle à une optique et/ou un moyen d'éclairage et/ou un canal de travail de l'endoscope.

9. Endoscope (1) avec une tige flexible (12) qui présente en particulier une section de déflexion (10) et un nombre de canaux de fonction et d'alimentation (6) qui peuvent être raccordés à une station de fonctionnement, **caractérisé par** une tête d'endoscope (2) du type élément rapporté, intégré ou adaptatif selon l'une quelconque des revendications 1 à 8.

10. Endoscope (1) selon la revendication 9, **caractérisé par** au moins un engrenage de rotation (11) pour la rotation d'une section distale de l'endoscope (1) ou de la tête d'endoscope (2) autour de son axe longitudinal.
